# EUROPEAN PATENT APPLICATION

(11) **EP 2 018 854 A1**
(43) Date of publication of application: **28.01.2009**
(21) Application number: 07252976.1
(22) Date of filing: 27.07.2007
(51) Int. Cl.: A61K 31/015, A61P 25/00

(54) **Novel combinations of neramexane for the treatment of neurodegenerative disorders**

(71) Applicant: Merz Pharma GmbH & Co. KGaA, 60318 Frankfurt am Main (DE)
(72) Inventor: Hanschmann, Angelika, 60388 Frankfurt am Main (DE); Althaus, Michael, 63067 Offenbach (DE); Gebauer, Alexander, 65191 Wiesbaden (DE); Parsons, Christopher, Graham, Raphael, 61130 Nidderau (DE); Danysz, Wojciech, 61130 Nidderau (DE)
(74) Representative: Goddard, Christopher Robert

(57) **Abstract**

The present invention relates to combinations comprising neramexane and a Glutamate Release Inhibitor (GRI) and the use of such combinations in the treatment of neurodegenerative disorders.

## Description

### FIELD OF THE INVENTION

The present invention relates to combinations comprising neramexane and a Glutamate Release Inhibitor (GRI) and the use of such combinations in the treatment of neurodegenerative disorders.

### BACKGROUND OF THE INVENTION

Glutamate is the main excitatory neurotransmitter in the CNS and glutamatergic dysfunction is involved in acute neurodegeneration (e.g. stroke and trauma), chronic neurodegeneration (e.g. Parkinson's disease, Alzheimer's disease, Huntington's disease, ALS) and in the symptomatology of numerous neurological and psychiatric disorders (e.g. epilepsy, Parkinson's disease, drug dependence, depression, anxiety and chronic pain).

Excitotoxicity contributes to neuronal degeneration in many CNS diseases, including ischaemia, trauma, and epilepsy, as well as chronic diseases, such as Alzheimer's disease (AD), Parkinson's disease and amyotrophic lateral sclerosis (ALS). Glutamate activates postsynaptic receptors, including the ionotropic N-methyl-D-aspartate (NMDA) receptor. It has been hypothesized that overactivation of NMDA receptors plays a pivotal role in the development of neurodegenerative disorders [Krieger et al., C. Trends Pharmacol Sci, 1996, 17, 114-120; Danysz et al., Neurotox Res, 2002, 4, 119-126; Arundine and Tymianski, Cell Calcium, 2004, 34, 325-337].

Motor Neuron Diseases are neurodegenerative disorders which involve a progressive loss of motor neurons. Examples of Motor Neuron Diseases include amyotrophic lateral sclerosis (ALS), virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy.

The use of Glutamate Release Inhibitors (GRIs), such as riluzole, lamotrigine, fosphenytoin, gabapentin, pregabalin and lubeluzole, has been suggested as promising approach for the therapy of neurodegenerative diseases. GRls are thought to be effective in treating neurodegenerative diseases because of their mode of action, namely, the blockade of glutamate transmission before it becomes neurotoxic or impeding neuronal discharges during seizures [Benismon et al., N Engl J Med, 1994, 330, 585-591; Lacomblez et al., Lancet, 1996, 347, 1425-1431].

Amyotrophic lateral sclerosis (ALS) involves the upper and lower motor neurons. The disease occurs in sporadic and genetic forms, which are clinically indistinguishable. The mechanism underlying the characteristic selective degeneration and death of motor neurons in this common adult motor neuron disease is still unknown; however, one of the major hypotheses as to selective motor neuron death is glutamate mediated exictotoxicity [Rothstein, et al., Clin Neurosci, 1995, 3, 348-359].

Riluzole, also known as 2-amino-6-trifluromethoxybenzothiazole or 6-(trifluoromethoxy)benzothiazol-2-amine, is the only approved drug for the treatment of amytrophic lateral sclerosis (ALS) and is currently marketed as RILUTEK® by Sanofi Aventis. Riluzole demonstrates improvement in bulbar and limb function, but only a small, although statistically significant, improvement of survival time (i.e., up to 90 days as compared to placebo). Thus, a need exists for improved drug therapy for ALS.

About 20% of ALS cases are monogenic and autosomal dominant (familial ALS - fALS). The most common cause of fALS are point mutations in the gene encoding superoxide dismutase1 (SOD1) an enzyme responsible for scavenging superoxide ions [Rosen et al., Nature. 1993; 362(6415):59-62].

The pathological hallmarks of ALS are degeneration of lower motor neurons in the brainstem and spinal cord, of upper motor neurons in the motor cortex and of the corticospinal tracts accompanied by reactive gliosis. The exact pathogenic mechanism underlying the selective motor neuron death in ALS has not been elucidated, although a number of possible mechanisms in sporadic and SOD1-linked ALS have been proposed [Van Damme et al., Neurodegenerative Dis. 2005;2:147-159].

Overexpression of SOD1 gene mutations in mice and rats mimics the clinical and pathological characteristics of ALS in humans, in which motor neurons degenerate and animals die shortly after onset of symptoms. High levels of mutant SOD1 RNA are required for development of ALS-like phenotypes within the short life span of mice. Experimental models of ALS play a pivotal role in understanding the pathogenesis of ALS and in testing new therapeutic interventions aimed at protecting against neurodegeneration [Gurney, N Engl J Med. 1994 Dec 22;331(25):1721-2; Dal Canto et al., Am J Pathol. 1994 Dec;145(6):1271-9]. The transgenic SOD1(G93A) mouse represents one of the most frequently used models for therapeutic interventions [Julien, Drug Discovery Today: Dis. Models 2007,doi:10.1016/j.ddmod.2006.12.003 (in press)].

The mechanisms and processes responsible for the selective loss of motor neurons are still unknown and may well be multifactorial. One of the major hypotheses for selective motor neuron death is glutamate mediated excitotoxicity [Rothstein et al., Ann. Neurol. 1990, 28, 18-25; Rothstein, Clinical Neuroscience, 1995, 3, 348-359; Shaw et al., Neurol, 1997, 244, S3-S14]. Excitotoxicity describes neuronal degeneration induced by overstimulation of glutamate receptors, released from presynaptic terminals. For detailed characterization of excitotoxic mechanisms and their relevance to ALS see Van Damme et al., Neurodegenererative Dis, 2005, 2:147-159.

In recent years, ALS research has been aimed at the development of anti-excitotoxic compounds, such as Riluzole which interferes with excitatory neurotransmission [Ludolph et al., J Neural Transm, 1999 [Suppl] 55:79-95].

Excessive activation of N-methyl-D-aspartate (NMDA) receptors appears to be a major mechanism of neuronal degeneration in a wide spectrum of neurological disorders [Lipton, et al., N Engl J Med, 1994 Mar 3, 330(9):613-22].

Memantine, a noncompetitive NMDA receptor antagonist, has shown positive effects in the SOD1(G93A) ALS-mouse model regarding disease progression and survival [Wang et al., Eur J Neurosci, 2005 Nov, 22(9):2376-80].

Neramexane, also known as 1-amino-1,3,3,5,5-pentamethylcyclohexane, is a member of the class of orally active 1-aminocyclohexanes, and has been found to be useful in the therapy of various diseases especially in certain neurological diseases, including Alzheimer's disease and neuropathic pain. It is believed that the therapeutic action of neramexane is related to the inhibition of the effects of excessive glutamate at the NMDA receptors of nerve cells, for which reason the compound is also categorized as an NMDA antagonist, or NMDA receptor antagonist. More specifically, neramexane appears to be a low to moderate-affinity, non-competitive NMDA-receptor antagonist believed to selectively block the excitotoxic effects associated with abnormal transmission of glutamate.

### SUMMARY OF THE INVENTION

The present invention relates to combinations comprising neramexane and a Glutamate Release Inhibitor (GRI) and the use of such combinations in the treatment of neurodegenerative disorders.

A further aspect of the invention relates to combinations comprising neramexane and a Glutamate Release Inhibitor (GRI) and the use of such combinations in the treatment of motor neuron diseases, including amyotrophic lateral sclerosis (ALS), virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy

A further aspect of the invention relates to combinations comprising neramexane and a Glutamate Release Inhibitor (GRI) selected from riluzole, RP66055, gabapentin, pregabalin, lamotrigine, lubeluzole, fosphenytoin, sipatrigine, MS-153, and FP-0011, and the use of such combinations in the treatment of motor neuron diseases, including amyotrophic lateral sclerosis (ALS), virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy

A further aspect of the invention relates to combinations comprising neramexane and a Glutamate Release Inhibitor (GRI) selected from riluzole, RP66055, gabapentin, pregabalin, lamotrigine, lubeluzole, fosphenytoin, sipatrigine, MS-153, and FP-0011, and the use of such combinations in the treatment of amyotrophic lateral sclerosis (ALS).

A further aspect of the invention relates to combinations comprising neramexane and riluzole as well as a method of treating an individual diagnosed with amyotrophic lateral sclerosis (ALS), comprising administering to the individual an effective amount of a combination of neramexane and riluzole.

A further aspect of the invention relates to the use of a combination comprising neramexane and a Glutamate Release Inhibitor (GRI) for the manufacture of a medicament for the treatment of neurodegenerative disorders.

A further aspect of the invention relates to the use of a combination comprising neramexane and a Glutamate Release Inhibitor (GRI) for the manufacture of a medicament for the treatment of motor neuron diseases, including amyotrophic lateral sclerosis (ALS), virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy.

A further aspect of the invention relates to the use of a combination comprising neramexane and a Glutamate Release Inhibitor (GRI) selected from riluzole, RP66055, gabapentin, pregabalin, lamotrigine, lubeluzole, fosphenytoin, sipatrigine, MS-153, and FP-0011 for the manufacture of a medicament for the treatment of motor neuron diseases, including amyotrophic lateral sclerosis (ALS), virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy.

A further aspect of the invention relates to the use of a combination comprising neramexane and a Glutamate Release Inhibitor (GRI) selected from riluzole, RP66055, gabapentin, pregabalin, lamotrigine, lubeluzole, fosphenytoin, sipatrigine, MS-153, and FP-0011 for the manufacture of a medicament for the treatment of amyotrophic lateral sclerosis (ALS).

A further aspect of the invention relates to the use of a combination comprising neramexane and riluzole for the manufacture of a medicament for treatment of an individual diagnosed with amyotrophic lateral sclerosis (ALS).

An additional aspect of the invention relates to a pharmaceutical composition for the treatment of neurodegenerative disorders comprising a therapeutically effective amount of a combination of neramexane and a Glutamate Release Inhibitor (GRI), and at least one pharmaceutically acceptable excipient.

A further aspect of the invention relates to a pharmaceutical composition for the treatment of motor neuron diseases, including amyotrophic lateral sclerosis (ALS), virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy, comprising a therapeutically effective amount of a combination of neramexane and a Glutamate Release Inhibitor (GRI), including riluzole, RP66055, gabapentin, pregabalin, lamotrigine, lubeluzole, fosphenytoin, sipatrigine, MS-153, and FP-0011, and at least one pharmaceutically acceptable excipient.

A further aspect of the invention relates to a pharmaceutical composition for the treatment of amyotrophic lateral sclerosis (ALS) comprising a therapeutically effective amount of a combination comprising neramexane and riluzole, and at least one pharmaceutically acceptable carrier or excipient.

A further aspect of the invention relates to a pharmaceutical composition for the treatment of amyotrophic lateral sclerosis (ALS) comprising a therapeutically effective amount of a combination comprising neramexane and riluzole in an immediate or modified release formulation.

### DETAILED DESCRIPTION OF THE INVENTION

The term "combination" applied to active ingredients is used herein to define a single pharmaceutical composition (formulation) comprising two active agents (e.g., a pharmaceutical composition comprising neramexane and riluzole) or two separate pharmaceutical compositions, each comprising an active agent (e.g. a pharmaceutical composition comprising neramexane or riluzole), to be administered conjointly.

Within the meaning of the present invention, the term "conjoint administration" is used to refer to administration of neramexane and a GRI (for example, riluzole) simultaneously in one composition, or simultaneously in different compositions, or sequentially. For the sequential administration to be considered "conjoint", however, neramexane and the second active agent must be administered separated by a time interval which still permits the resultant beneficial effect for treating a neurodegenerative disorder (such as amyotrophic lateral sclerosis (ALS)) in a mammal.

The term "treat" is used herein to mean to relieve or alleviate at least one symptom of a disease in a subject. Within the meaning of the present invention, the term "treat" also denotes to arrest, delay the onset (i.e., the period prior to clinical manifestation of a disease) and/or reduce the risk of developing or worsening a disease.

As used herein, the term motor neuron diseases includes amyotrophic lateral sclerosis (ALS), virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy.

Neramexane (1-amino-1,3,3,5,5-pentamethylcyclohexane) is disclosed in U.S. Patent Nos. 6,034,134 and 6,071,966, the subject matter of which patents is hereby incorporated by reference. Neramexane, may be used according to the invention in the form of any of its pharmaceutically acceptable salts, solvates, isomers, conjugates, prodrugs, metabolites, and derivatives, any references to neramexane in this description should be understood as also referring to such salts, solvates, isomers, conjugates, prodrugs, metabolites, and derivatives.

As used herein, the term Glutamate Release Inhibitor (GRI) includes riluzole, RP66055, gabapentin, pregabalin, lamotrigine, lubeluzole, fosphenytoin, sipatrigine, MS-153, and FP-0011.

Riluzole (2-amino-6-trifluromethoxybenzothiazole or 6-(trifluoromethoxy)benzothiazol-2-amine) is disclosed in US Patent No. 4,370,338, the subject matter of which is hereby incorporated by reference. Riluzole, may be used according to the invention in the form of any of its pharmaceutically acceptable salts, solvates, isomers, conjugates, prodrugs, metabolites, and derivatives, any references to riluzole in this description should be understood as also referring to such salts, solvates, isomers, conjugates, prodrugs, metabolites, and derivatives.

RP66055 (3-{2-[1-(4-fluorophenyl-piperzinyl)]ethyl}-2-imino-6-trifluoromethoxybenzothiazoline) is disclosed in Jimonet, et, al. Bioorg Med Chem, 1994 2: 793-8. RP66055 may be used according to the invention in the form of any of its pharmaceutically acceptable salts, solvates, isomers, conjugates, prodrugs, metabolites, and derivatives, any references to RP66055 in this description should be understood as also referring to such salts, solvates, isomers, conjugates, prodrugs, metabolites, and derivatives.

Gabapentin (2-[1-(aminomethyl)cyclohexyl]acetic acid) may be used according to the invention in the form of any of its pharmaceutically acceptable salts, solvates, isomers, conjugates, prodrugs, metabolites, and derivatives, any references to gabapentin in this description should be understood as also referring to such salts, solvates, isomers, conjugates, prodrugs, metabolites, and derivatives.

Pregabalin ((S)-3-(aminomethyl)-5-methylhexanoic acid) may be used according to the invention in the form of any of its pharmaceutically acceptable salts, solvates, isomers, conjugates, prodrugs, metabolites, and derivatives, any references to pregabalin in this description should be understood as also referring to such salts, solvates, isomers, conjugates, prodrugs, metabolites, and derivatives.

Lamotrigine (6-(2,3-dichlorophenyl)-1,2,4-triazine-3,5-diamine) may be used according to the invention in the form of any of its pharmaceutically acceptable salts, solvates, isomers, conjugates, prodrugs, metabolites, and derivatives, any references to lamotrigine in this description should be understood as also referring to such salts, solvates, isomers, conjugates, prodrugs, metabolites, and derivatives.

Lubeluzole ((S)-4-[(2-benzothiazolyl)methylamino]-α-[(3,4-difluoro-phenoxy)methyl]-1-piperidineethanol) is disclosed in US Patent No. 5,434,168. Lubeluzole may be used according to the invention in the form of any of its pharmaceutically acceptable salts, solvates, isomers, conjugates, prodrugs, metabolites, and derivatives, any references to lubeluzole in this description should be understood as also referring to such salts, solvates, isomers, conjugates, prodrugs, metabolites, and derivatives.

Fosphenytoin (2,5-dioxo-4,4-dipenyl-imidazolidin-1-yl)methoxyphosphonic acid) may be used according to the invention in the form of any of its pharmaceutically acceptable salts, solvates, isomers, conjugates, prodrugs, metabolites, and derivatives, any references to fosphenytoin in this description should be understood as also referring to such salts, solvates, isomers, conjugates, prodrugs, metabolites, and derivatives.

Sipatrigine (2-(4-methylpiperazin-1-yl)-5-(2,3,5-trichlorophenyl)pyrimidin-4-amine) may be used according to the invention in the form of any of its pharmaceutically acceptable salts, solvates, isomers, conjugates, prodrugs, metabolites, and derivatives, any references to sipatrigine in this description should be understood as also referring to such salts, solvates, isomers, conjugates, prodrugs, metabolites, and derivatives.

MS-153 ((R)-(-)-5-methyl-1-nicotinoyl-2-pyrazoline) may be used according to the invention in the form of any of its pharmaceutically acceptable salts, solvates, isomers, conjugates, prodrugs, metabolites, and derivatives, any references to MS-153 in this description should be understood as also referring to such salts, solvates, isomers, conjugates, prodrugs, metabolites, and derivatives.

Pharmaceutically acceptable salts include, but are not limited to, acid addition salts, such as those made with hydrochloric, methylsulfonic, hydrobromic, hydroiodic, perchloric, sulfuric, nitric, phosphoric, acetic, propionic, glycolic, lactic, pyruvic, malonic, succinic, fumaric, tartaric, citric, benzoic, carbonic, cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid. All of these salts (or other similar salts) may be prepared by conventional means. The nature of the salt is not critical, provided that it is non-toxic and does not substantially interfere with the desired pharmacological activity.

The term "analog" or "derivative" is used herein in the conventional pharmaceutical sense, to refer to a molecule that structurally resembles a reference molecule (such as neramexane or riluzole), but has been modified in a targeted and controlled manner to replace one or more specific substituents of the referent molecule with an alternate substituent, thereby generating a molecule which is structurally similar to the reference molecule. Synthesis and screening of analogs (*e*.*g*., using structural and/or biochemical analysis), to identify slightly modified versions of a known compound which may have improved or biased traits (such as higher potency and/or selectivity at a specific targeted receptor type, greater ability to penetrate mammalian blood-brain barriers, fewer side effects, etc.) is a drug design approach that is well known in pharmaceutical chemistry.

The term "therapeutically effective" applied to dose or amount refers to that quantity of a compound or pharmaceutical composition that is sufficient to result in a desired activity upon administration to a mammal in need thereof.

The term "sub-threshold" refers to the amount of an active ingredient inadequate to produce a response, *i*.*e*., an amount below the minimum effective amount when the active ingredient is used as monotherapy.

The term "sub-optimal" in the same context means an amount of an active ingredient that produces a response but not to its full extent, which would be achieved with a higher amount.

The term "additive" refers to the combined effect of administering two compounds, where the overall response is equal to, or nearly equal to the sum of the responses if the compounds were administered as monotherapy.

The term "synergistic" refers to the combined effect of administering two therapeutic compounds where the overall response is greater than the sum of the two individual effects. The term synergy also refers to the combined effect of administering an amount of one compound that, when administered as monotherapy, produces no measurable response but, when administered in combination with another therapeutic compound, produces an overall response that is greater than that produced by the second compound alone.

The phrase "pharmaceutically acceptable", as used in connection with compositions of the invention, refers to molecular entities and other ingredients of such compositions that are physiologically tolerable and do not typically produce untoward reactions when administered to a mammal (*e*.*g*., human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical compositions of the invention refers to a diluent, excipient, or vehicle with which an active compound (*e*.*g*., neramexane) is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by A.R. Gennaro, 20th Edition.

The term "about" or "approximately" usually means within 20%, alternatively within 10%, including within 5% of a given value or range. Alternatively, especially in biological systems, the term "about" means within about a log (*i*.*e*., an order of magnitude), including within a factor of two of a given value.

In conjunction with the methods of the present invention, also provided are pharmaceutical compositions comprising a therapeutically effective amount of neramexane and/or a therapeutically acceptable amount of a GRI (for example, riluzole). The compositions of the invention may further comprise a carrier or excipient (all pharmaceutically acceptable). The compositions may be formulated for once-a-day administration, twice-a-day administration, or three times a day administration.

The composition or a single active ingredient of the present invention may be used for the manufacture of a medicament for the treatment of one of the mentioned disorders, wherein the medicament is adapted to or appropriately prepared for a specific administration as disclosed herein (e.g., to once-a-day, twice-a-day, or three times a day administration). For this purpose the package leaflet and/or the patient information contains corresponding information.

According to the present invention, the dosage form of the compositions may be a solid, semisolid, or liquid formulation according to the following.

The compositions may be administered orally, topically, parenterally, or mucosally (e.g., buccally, by inhalation, or rectally) in dosage unit formulations containing conventional non-toxic pharmaceutically acceptable carriers. The compositions may be administered orally in the form of a capsule, a tablet, or the like, or as a semi-solid, or liquid formulation (see Remington's Pharmaceutical Sciences, 20th Edition, by A.R. Gennaro).

For oral administration in the form of a tablet or capsule, the compositions may be combined with a non-toxic, pharmaceutically acceptable excipients such as binding agents (e.g., pregelatinized maize starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talc, or silica, steric acid, sodium stearyl fumarate, glyceryl behenate, calcium stearate, and the like); disintegrants (e.g., potato starch or sodium starch glycolate); or wetting agents (e.g., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like.

The tablets may be coated with a concentrated sugar solution which may contain e.g., gum arabic, gelatine, talcum, titanium dioxide, and the like. Alternatively, the tablets can be coated with a polymer that dissolves in a readily volatile organic solvent or mixture of organic solvents. In specific embodiments, neramexane is formulated in to immediate-release (IR) or modified-release (MR) tablets. Immediate release solid dosage forms permit the release of most or all of the active ingredient over a short period of time, such as 60 minutes or less, and make rapid absorption of the drug possible (for example, immediate release formulations of neramexane are disclosed in US Published Application Nos. 2006/0002999 and 2006/0198884, the subject matter of which is hereby incorporated by reference). Modified release solid oral dosage forms permit the sustained release of the active ingredient over an extended period of time in an effort to maintain therapeutically effective plasma levels over similarly extended time intervals and/or to modify other pharmacokinetic properties of the active ingredient (for example, modified release formulations of neramexane are disclosed in US Published Application No. 2007/0141148, the subject matter of which is hereby incorporated by reference).

For the formulation of soft gelatin capsules, the active substances may be admixed with e.g., a vegetable oil or poly-ethylene glycol. Hard gelatin capsules may contain granules of the active substances using either the above mentioned excipients for tablets e.g., lactose, saccharose, sorbitol, mannitol, starches (e.g., potato starch, corn starch or amylopectin), cellulose derivatives or gelatine. Also liquids or semisolids of the drug can be filled into hard gelatine capsules.

The compositions of the invention can also be introduced in microspheres or microcapsules, e.g., fabricated from polyglycolic acid/lactic acid (PGLA) (see, e.g., U.S. Patents No. 5,814,344; 5,100,669 and 4,849,222; PCT Publications No. WO 95/11010 and WO 93/07861). Biocompatible polymers may be used in achieving controlled release of a drug, include for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

Formulation of the compositions of the invention in a semi-solid or liquid form may also be used. The active ingredient (i.e., neramexane and/or, for example, riluzole) may constitute between 0.1 and 99% by weight of the formulation, more specifically between 0.5 and 20% by weight for formulations intended for injection and between 0.2 and 50% by weight for formulations suitable for oral administration.

In one embodiment of the invention, the compositions are administered in modified release formulations. Modified release dosage forms provide a means for improving patient compliance and for ensuring effective and safe therapy by reducing the incidence of adverse drug reactions. Compared to immediate release dosage forms, modified release dosage forms can be used to prolong pharmacologic action after administration, and to reduce variability in the plasma concentration of a drug throughout the dosage interval, thereby eliminating or reducing sharp peaks.

A modified release form dosage may comprise a core either coated with or containing a drug. The core being is then coated with a release modifying polymer within which the drug is dispersed. The release modifying polymer disintegrates gradually, releasing the drug over time. Thus, the outer-most layer of the composition effectively slows down and thereby regulates the diffusion of the drug across the coating layer when the composition is exposed to an aqueous environment, i.e. the gastrointestinal tract. The net rate of diffusion of the drug is mainly dependent on the ability of the gastric fluid to penetrate the coating layer or matrix and on the solubility of the drug itself.

In another embodiment of the invention, the compositions are formulated in oral, liquid formulations. Liquid preparations for oral administration can take the form of, for example, solutions, syrups, emulsions or suspensions, or they can be presented as a dry product for reconstitution with water or other suitable vehicle before use. Preparations for oral administration can be suitably formulated to give controlled or postponed release of the active compound. For example, oral liquid formulations of neramexane are described in PCT International Application No. PCT/US2004/037026, the subject matter of which is hereby incorporated by reference.

For oral administration in liquid form, the compositions may be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), nonaqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate, citric acid) can also be added to stabilize the dosage forms. For example, solutions may contain from about 0.2% to about 20% by weight of neramexane, with the balance being sugar and mixture of ethanol, water, glycerol and propylene glycol. Optionally, such liquid formulations may contain coloring agents, flavoring agents, saccharine and carboxymethyl-cellulose as a thickening agent or other excipients.

In another embodiment, a therapeutically effective amount of the active substance is administered in an oral solution containing a preservative, a sweetener, a solubilizer, and a solvent. The oral solution may include one or more buffers, flavorings, or additional excipients. In a further embodiment, a peppermint or other flavoring is added to the oral liquid formulation.

For administration by inhalation, the compositions may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

Solutions for parenteral applications by injection may be prepared in an aqueous solution of a water-soluble pharmaceutically acceptable salt of the active substances, preferably in a concentration of from about 0.5% to about 10% by weight. These solutions may also contain stabilizing agents and/or buffering agents and may conveniently be provided in various dosage unit ampoules.

Dosage units for rectal application may be solutions or suspensions or may be prepared in the form of suppositories or retention enemas comprising neramexane in a mixture with a neutral fatty base, or gelatin rectal capsules comprising the active substances in admixture with vegetable oil or paraffin oil.

The formulations of the invention may be delivered parenterally, i.e., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, e.g., in ampoules or in multi-dose containers, with an added preservative. Alternatively, the active ingredient may be in powder form for reconstitution with a suitable vehicle, e.g., sterile pyrogen-free water, before use.

The invention also provides a pharmaceutical pack or kit comprising one or more containers containing the active substances (i.e., neramexane and/or, for example, riluzole) and, optionally, more of the ingredients of the formulation. In a specific embodiment, the compositions are provided as oral solutions (2 mg/ml) for administration with the use of a 2 teaspoon capacity syringe (dosage KORC®). Each oral syringe has blue hatch marks for measurement, with lines on the right side of the syringe (tip down) representing tsp units, and those on the left representing ml units.

The optimal therapeutically effective amount of the components of the compositions of the present invention may be determined experimentally, taking into consideration the exact mode of administration, from in which the drug is administered, the indication toward which the administration is directed, the subject involved (e.g., body weight, health, age, sex, etc.), and the preference and experience of the physician or veterinarian in charge.

Toxicity and therapeutic efficacy of the compositions of the invention may be determined by standard pharmaceutical procedures in experimental animals, e.g., by determining the LD₅₀ (the dose lethal to 50% of the population) and the ED₅₀ (the dose therapeutically effective in 50% of the population). The dose ratio between therapeutic and toxic effects is the therapeutic index and it may be expressed as the ratio LD₅₀/ED₅₀. Compositions that exhibit large therapeutic indices are preferred.

Suitable daily doses of the active compounds of the invention in therapeutic treatment of humans are about 0.01-10 mg/kg bodyweight on peroral administration and 0.001-10 mg/kg bodyweight on parenteral administration. Suitable daily doses for neramexane (e.g., neramexane mesylate) are within the range from about 5 mg to about 150 mg per day, such as from about 5 mg to about 120 mg, from about 5 mg to about 100 mg, or from about 5 mg to about 50 mg per day. Suitable daily doses for neramexane (e.g., neramexane mesylate) are 6.25 mg, 12.5 mg, 25 mg, 50 mg, or 75 mg per day. Suitable daily doses for riluzole are within the range from about 5 mg to about 150 mg per day, such as from about 5 mg to about 100 mg per day, such as from about 50 mg to about 100mg per day, such as 50 mg twice a day

### EXAMPLES

The following examples illustrate the invention without limiting its scope.

### EXAMPLE 1: Effects of Neramexane in combination with Riluzole in an ALS mouse model

Overexpression of SOD1 gene mutations in mice and rats mimics the clinical and pathological characteristics of ALS in humans, in which motor neurons degenerate and animals die shortly after onset of symptoms. The transgenic SOD1(G93A) mouse represents one of the most frequently used models for evaluating therapeutic interventions.

The mouse model used in this study is characterized by overexpression of the human mutated enzyme Cu/Zn SOD1 bearing a missence mutation from Glycine to Alanine at position 93 (G93A). mRNA-levels of mutated SOD1 are 40-fold the normal mRNA levels of endogenous mouse SOD1 [Gurney M. E., J. Neurol. Sci., 1997, 152 Suppl 1, S67-73.]

Lifespan of transgenic mice bearing a high copy number of human, mutated SOD1(G93A) is about 130 days [Gumey M. E., J. Neurol. Sci., 1997, 152 Suppl 1, S67-73], disease onset arrives about the age of 90 days and at the age of 110 days mice develop paresis of one or both hind limbs [Wang et al., Eur J Neurosci. 2005 Nov;22(9):2376-80].

Neramexane is tested for the purpose of amelioration of ALS pathogenesis in the SOD1 (G93A) transgenic mouse model of ALS. The efficacy of Neramexane is compared to the efficacy of Riluzole and Memantine, which has been shown to exhibit positive effects in the ALS mouse model [Wang et al., Eur J Neurosci. 2005 Nov;22(9):2376-80]. Combination treatment with Riluzole and Neramexane is also tested.

### Materials and Methods

### Animals

For this study the SOD1-G93A mouse model is used. This model is well established for investigations regarding Motor Neuron Disease (MND). The mice are characterized by overexpression of the human mutated enzyme Cu/Zn SOD1, showing a missence mutation from Glycine to Alanine at position 93 (G93A) and serve as a mouse model for the familial form of amyotrophic lateral sclerosis (ALS). The specific name of the strain is B6SJL-TgN(SOD1-G93A)1 Gur/J.

Mice are caged separately with access to a running wheel at the age of 38 days and are allowed to acclimatise for 7 days before the start of the study. Drug treatment starts at day 45. There is automatic control of light cycle, temperature and humidity. Light hours are 5:00 am - 6:30 pm. Daily monitoring indicates that temperature and humidity remain within the target ranges of 20 °C ± 3°C and 80 ± 10%, respectively. The animals are housed in polypropylene cages (ca 26 x 20 x 14 cm, with mesh tops from arrival, 1 per cage). Cages, bedding, and water bottles are changed at regular intervals, i.e. every 7 days. Standard Diet (ssniff® MZ Ereich) is available to the animals ad libitum. The animals have access to domestic quality mains water ad libitum. To monitor activity levels, running wheels are made available to the animals.

### Drugs

Neramexane is supplied by MERZ pharmaceuticals as neramexane mesylate. Memantine is supplied by MERZ pharmaceuticals. Riluzole is supplied by Sequoia Research Products. Riluzole and neramexane are administered orally. Drugs are dissolved in drinking water. Calculation is based on an average consumption of 6mL/24 h. Memantine is administered twice a day subcutaneously. For all drug-preparation an average adult weight of 19g (female mice) and 24 g (male mice) is assumed.

### Treatment

As soon as results of genotyping are available (age of ca 30 days) transgenic mice are randomly allocated to treatment groups, such that the treatment groups are evenly distributed throughout the caging system.

The treatment groups and animal numbers are arranged as follows:

| **Group** | **Treatment Group** | **Dose Level (mg.kg⁻¹.day⁻¹)*** | Number **of Animals** |
|---|---|---|---|
| **1** | Vehicle Control | 0 | Male mice n=12 |
| | | | Female mice n=12 |
| **2** | Riluzole | 35 | Male mice n=12 |
| | | | Female mice n=12 |
| **3** | Memantine | 20 | Male mice n=12 |
| | | | Female mice n=12 |
| **4** | Neramexane | 50 | Male mice n=16 |
| | Neramexane | 150 | Female mice n=16 |
| **5** | Neramexane + Riluzole | 50 + 35 | Male mice n=16 |
| | Neramexane + Riluzole | 150 + 35 | Female mice n=16 |

*Doses refer to respective salts*

Treatment starts at the age of 45 days and stops at disease end-stage. Animals of treatment group 3 (Memantine) are dosed subcutaneously twice a day at a constant dose volume of each with 100 µl, using a steel dosing cannula. The concentration of the solution is determined by the average adult weight of the male (24 g) and female (19 g) animals. Animals of treatment groups 2, 4 and 5 are dosed with drugs (Riluzole, Neramexane and Neramexane plus Riluzole in combination) dissolved in drinking water. The concentration of the solutions is determined by the average adult weight of male (24 g) and female (19 g) animals and by an average consumption of 6 ml drinking-water. Consumption of drinking water is checked randomly.

### Evaluation of Data

To measure the therapeutic efficacy of the drugs four parameters are analyzed for each treatment group.
1. Age of Disease Onset
2. Age of First Paresis
3. Age of Disease final stage (survival)
4. Motor Performance in the running wheel

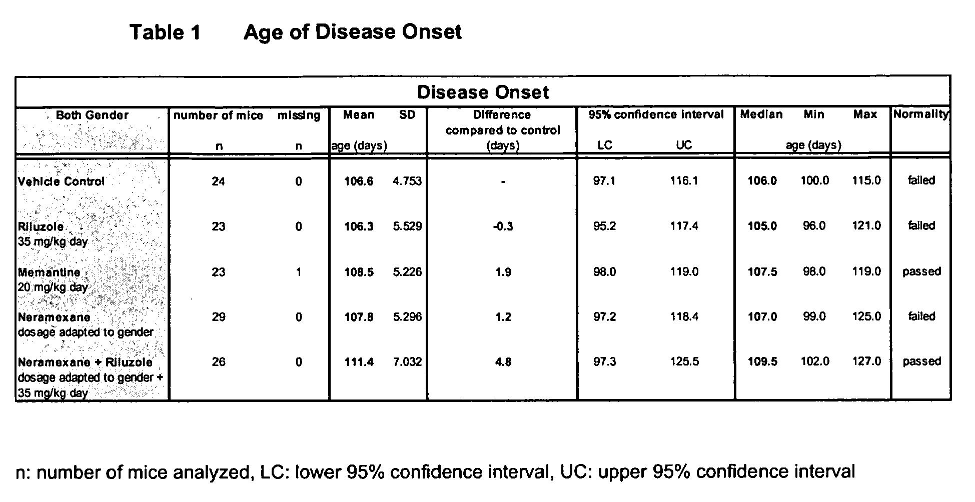

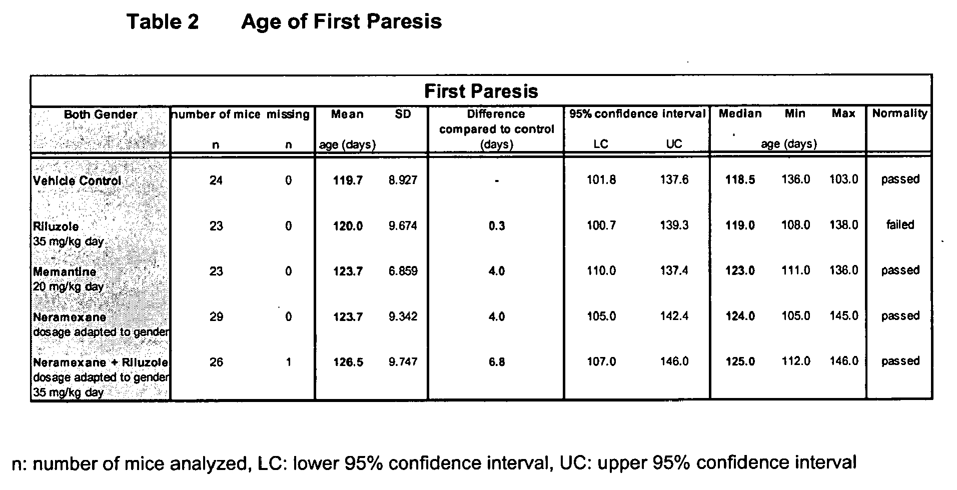

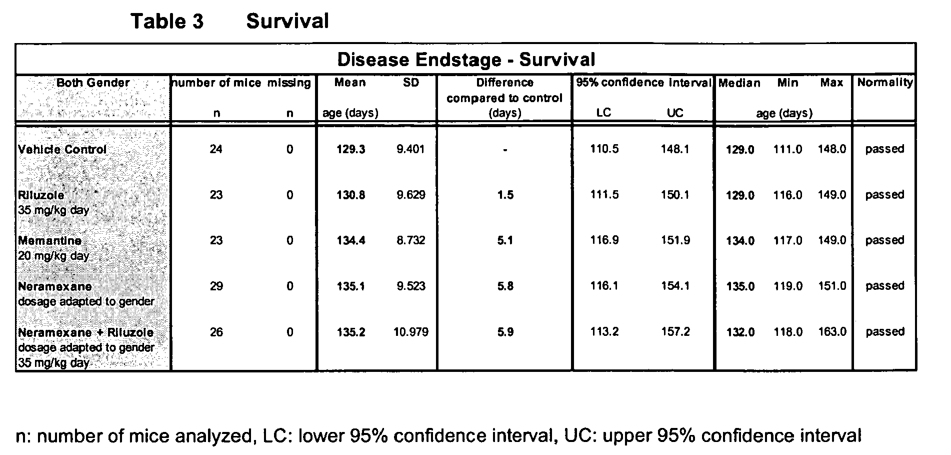

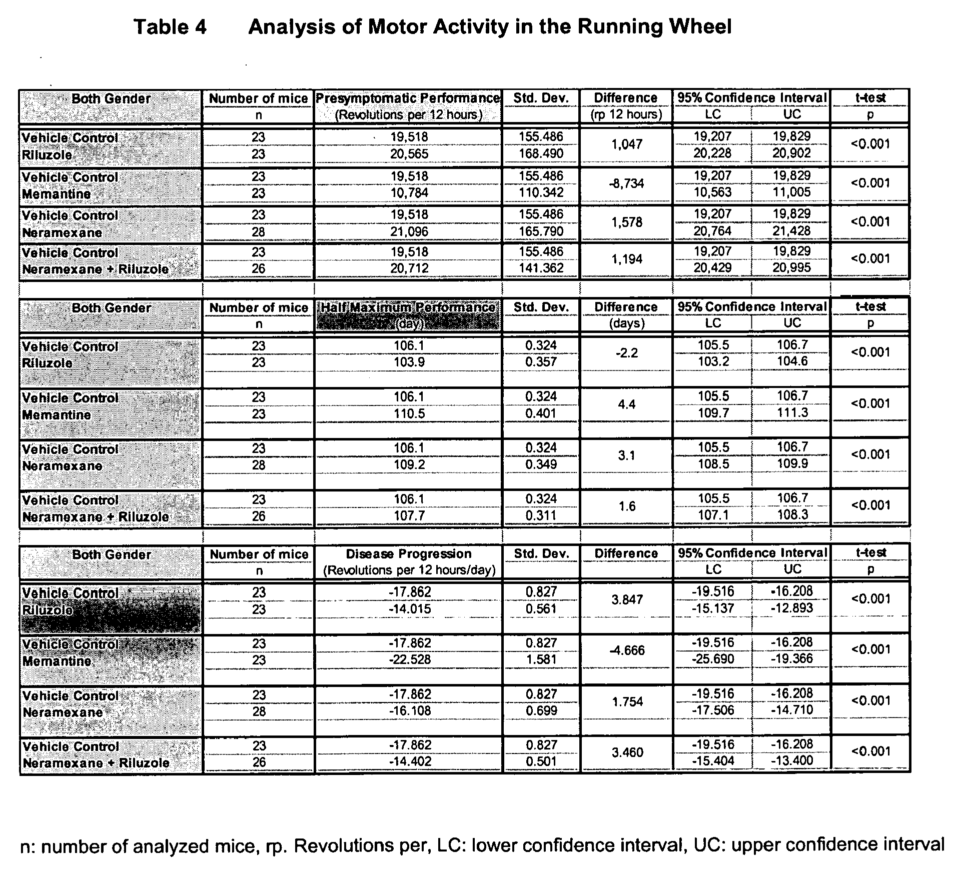

### Results

The most effective treatment with respect to the three parameters which indicate delayed disease progression (age of disease onset, age of first paresis and survival) occurs in the combination group (i.e., oral administration of Neramexane and Riluzole), with a delay of 5 to 7 days, and statistically significant differences regarding age of onset and age of first paresis. Motor activity analysis indicates no evidence for delayed disease progression in any of the test groups.

### EXAMPLE 2: Evaluation of Neramexane in combination with Riluzole for the treatment of amyotrophic lateral sclerosis (ALS)

The objective of this pilot project is to conduct a clinical trial to assess the efficacy of a combination comprising neramexane and riluzole as a treatment for amyotrophic lateral sclerosis (ALS). ALS patients treated with a combination comprising neramexane and riluzole may be expected to demonstrate a reduction in disease progression which is substantially greater than the reduction in disease progression provided by riluzole monotherapy.

### Study Design

The primary objective of this study is to investigate the effect of neramexane as well as the effect of neramexane in combination with riluzole on disease progression (as measured by ALS-FRS-r) in patients suffering from ALS.

An adaptive design approach is used in this study. A formal sample size estimation is performed on the basis of an unblinded interim analysis after treatment completion of a total of 150 patients. The unblinded interim analysis is performed by an Independent Data Monitoring Committee.

To ensure that the site of disease onset which is known to be an important covariate is equally distributed within both treatment groups the randomization is performed stratified according to this factor.

### Statistical Procedures and Populations for Analysis

In order to be eligible to participate in the study, patients must meet the following criteria:
- Signed informed consent
- Male or female patients aged ≥18 and ≤75 years with a diagnosis of laboratory-supported probable, probable, or definite ALS, according to modified El Escorial criteria, familial or sporadic form of ALS
- ALS-FRS-r at screening >33 and <43
- Disease progression measured by individual monthly decline in ALS-FRS-r score >0.4 and <1.2 (calculated from disease duration starting with first occurrence of pareses and bulbar symptoms and screening score)
- First diagnosis of pareses and bulbar symptoms > 9 months prior to baseline
- Vital capacity (VC) > 70% of age-based normal value
- Patient is expected to understand the nature of the study, potential risks or discomfort, and to comply with the scheduled visits and dosing scheme
- For patients on riluzole therapy: stable dose of 100 mg per day for at least 6 weeks prior to baseline
- For women with childbearing potential (last menstruation less than 1 year prior to screening): negative pregnancy test at baseline, must be using an adequate birth control method

Patients meeting any of the following criteria are excluded from the study:
- Requirement for invasive ventilation or tracheotomy
- Non-invasive ventilation
- Percutane endoscopic gastrostomy (PEG) at Screening
- Diagnosis of other neurodegenerative diseases, e.g. Parkinson's disease, Alzheimer's disease, etc.
- Monoclonal gammopathy with an associated hematological malignancy
- BMI < 18
- Patients with clinically relevant abnormal ECG findings
- Patients who have a clinically relevant neurological or psychiatric disorder other than ALS or other severe or uncontrolled systemic diseases (e.g. cardiovascular (including rhythm disorder), renal, pulmonary, hepatic, endocrinal, hematological, or gastrointestinal) which might interfere with the trial
- ALS with frontotemporal dementia
- Patients with systolic blood pressure greater than 160 mm Hg or less than 90 mm Hg or diastolic blood pressure greater than 100 mm Hg or less than 50 mm Hg at the screening or baseline visit with or without stable antihypertensive medication
- Deep vein thrombosis and pulmonary embolism
- Patients with orthostatic dysregulation
- AST or ALT > 2.5 times upper normal limit
- Known renal insufficiency
- Creatinine > 1.5 times upper normal limit
- Clinical significant disturbances of absorption in the upper gastrointestinal tract
- Use of NMDA antagonists (memantine, dextromethorphan, ketamine) or cannabinoids
- Alcohol, analgesic, or narcotic substance abuse during the last 6 months prior to baseline
- Patients who require concomitant therapy with any prohibited medication (see)
- Participation in any investigational drug study or use of any investigational drug within 3 months prior to baseline
- Patients who previously participated in this or any other Neramexane trial
- Patients with known allergy, hypersensitivity, or intolerance to Neramexane, amantadine, ketamine, memantine, or riluzole add further ingredients of study medication
- Nursing mother or pregnant woman, verified by a positive pregnancy test
- Evidence or suspicion that the patient might not comply with the study directives and/or that he/she is not reliable or trustworthy
- Evidence or suspicion that the patient is not willing or unable to understand the information that is given to him/her as part of the informed consent, in particular regarding the risks and discomfort to which he/she would agree to be exposed
- Employees or direct relatives of an employee of the CRO, the Study Center or Merz Pharmaceuticals

The scheduled visits for evaluation of each patient are as follows:

**Visit 1** (initial screening): After signing the consent form, the subject undergoes an evaluation, in which primary and secondary parameters are evaluated. Patient eligibility for study is evaluated via review of inclusion/exclusion criteria.

**Visit 2** (baseline): Subject is evaluated for study eligibility based on a review of the inclusion/exclusion criteria. Study procedures as well as concomitant medications are reviewed with the subject. Subject is enrolled in the study and medication is dispensed as described below.

**Visits 3-9:** These visits occur one, two, three, six, nine, twelve, and fifteen months after baseline. Review of concomitant medications as well as the occurrence of adverse events since the last visit are conducted with subject. Primary and secondary parameters are evaluated. Medication is dispensed as described below.

**Visit 10:** This visit occurs thirty-one days after the last dose. Change in baseline in ALS-FRS-r score as well as secondary parameters are evaluated.

### Administration of Neramexane/Riluzole combination

### Neramexane

Neramexane mesylate 25 mg modified release tablets and matching placebo tablets are administered as film coated tablets.

Neramexane (or placebo) is uptitrated to a maximum daily dose of 75 mg, starting with a daily dose of 25 mg for one week, and increasing dosage in 25 mg steps at weekly intervals.

The study drug is presented as three tablets per day (morning, noon, and evening). During Week 1 of the uptitration period, patients receive one neramexane tablet and two placebo tablets per day. During Week 2, patients receive two neramexane tablets and one placebo tablet per day. During Week 3 (and continuing through Month 15 of the study), patients receive three neramexane tablets or two neramexane tablets and one placebo tablet per day (depending on individual tolerability).

### Riluzole

Riluzole is administered as commercially available tablets containing 50 mg riluzole at a dose of 100 mg per day.

### Efficacy

Patients are evaluated using functional scales as well as quality of life scales.

Primary Outcome
- Change from baseline in ALS-FRS-r score after 15 months of double-blind treatment in the ITT subset

Secondary Outcomes
- Change from baseline in ALS-FRS-r score after 3 and 9 months of double-blind treatment
- Change in individual slope of disease progression (monthly decline in ALS-FRS-r from baseline to end of Month 15 of double-blind treatment as compared to screening value)
- Time to event (event defined as the occurrence of either death, tracheotomy, invasive ventilation, or non-invasive ventilation, also the occurrence of every single item)
- Number of patients with percutaneous endoscopic gastrostomy tube (PEG) after 15 months of double-blind treatment
- Change from baseline in duration of non-invasive ventilation per day after 3, 9, and 15 months of double-blind treatment
- Area under the curve of vital capacity (VC) expressed as percent of age norm between screening visit and end of Month 15 of double-blind treatment
- Area under the curve of forced vital capacity (FVC) expressed as percent of age norm between screening visit and end of Month 15 of double-blind treatment
- Change from baseline in muscle strength measured with Manual Muscle Testing (MMT) after 3, 9, and 15 months of double-blind treatment
- Change from baseline in the short form health survey (SF-36) after 9 and 15 months of double-blind treatment
- Time to onset of non-invasive ventilation
- Time to onset of percutaneous endoscopic gastrostomy tube (PEG)

### Data Analysis

The change from baseline in ALS-FRS-r score after 15 months of double-blind treatment (primary efficacy analysis) and during the course of the study (secondary efficacy parameter) is analyzed for the ITT subset using an ANCOVA approach with center, treatment group, and site of disease onset (bulbar or spinal) as factors and baseline value as covariate. The primary efficacy analysis is based only on patients who are on stable riluzole dose at baseline.

The change from baseline in duration of non-invasive ventilation is analyzed by using the same ANCOVA approach as described above but without extension of factors.

The change in individual slope of disease progression and the final MMT score is analyzed non-parametrically by using an ANCOVA approach on the ranked data with center, treatment group, and site of disease onset (bulbar or spinal) as factors and ranked slope at randomization or ranked MMT score at baseline as covariate, respectively.

The time to event curves are compared by treatment with a log-rank test and after adjustment for site of disease onset (bulbar or spinal) with a Cox proportional hazards model.

The number of patients with percutaneous endoscopic gastrostomy tube and the AUC of the vital capacity (VC) and the forced vital capacity (FVC) are analyzed with an ANCOVA approach with center, treatment group, and site of disease onset (bulbar or spinal) as factors.

The SF-36 scores are analyzed with an ANCOVA approach with center, treatment group, and site of disease onset (bulbar or spinal) as factors and the corresponding baseline value as covariate.

For all efficacy analyses the intent to treat principle is used taking into account all randomized patients with at least one intake of study medication (Neramexane or placebo) and one post-baseline measurement of the ALS-FRS-r score.

### Discussion

The neramexane/riluzole combination treated group demonstrates a reduction in disease progression which is substantially greater than the reduction in disease progression provided by treatment with riluzole and placebo.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

All patents, applications, publications, test methods, literature, and other materials cited herein are hereby incorporated by reference.

## Claims

1. Use of neramexane, or a pharmaceutically acceptable salt thereof, in the manufacture of a medicament for the treatment of neurodegenerative disorders in combination with a Glutamate Release Inhibitor.

2. Use as claimed in Claim 1, wherein the neurodegenerative disorder is a motor neuron disease.

3. Use as claimed in Claim 2, wherein the motor neuron disease is selected from amyotrophic lateral sclerosis, virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy.

4. Use as claimed in Claim 3, wherein the motor neuron disease is amyotrophic lateral sclerosis.

5. Use as claimed in any preceding claim, wherein neramexane is employed as its mesylate salt.

6. Use as claimed in any preceding claim, wherein the Glutamate Release Inhibitor is selected from riluzole, RP66055, gabapentin, pregabalin, lamotrigine, lubeluzole, fosphenytoin, sipatrigine, MS-153, and FP-0011 and pharmaceutically acceptable salts thereof.

7. Use as claimed in Claim 6, wherein the Glutamate Release Inhibitor is selected from riluzole and pharmaceutically acceptable salts thereof.

8. Use as claimed in any preceding claim, wherein the medicament is manufactured for simultaneous coadministration with the Glutamate Release Inhibitor.

9. Use as claimed in Claim 8, wherein the medicament is manufactured as a unitary dosage form comprising neramexane, or a pharmaceutically acceptable salt thereof, and the Glutamate Release Inhibitor.

10. Use as claimed in Claim 9, wherein the medicament is manufactured for administration at a dose of 5 to 150 mg/day for neramexane or a pharmaceutically acceptable salt thereof, and 5 to 150 mg/day for riluzole, or a pharmaceutically acceptable salt thereof.

11. Use as claimed in Claim 10, wherein the dose for neramexane, or a pharmaceutically acceptable salt thereof, is 5 mg to 100 mg/day, and the dose for riluzole, or a pharmaceutically acceptable salt thereof, is 5 mg to 100 mg/day.

12. Use as claimed in Claim 11, wherein the dose for neramexane, or a pharmaceutically acceptable salt thereof, is 5 mg to 75 mg/day, and the dose for riluzole, or a pharmaceutically acceptable salt thereof, is 5 mg to 100 mg/day.

13. Use as claimed in Claim 12, wherein the dose for neramexane, or a pharmaceutically acceptable salt thereof, is 25 mg/day, and the dose for riluzole, or a pharmaceutically acceptable salt thereof, is 100 mg/day.

14. Use as claimed in Claim 12, wherein the dose for neramexane, or a pharmaceutically acceptable salt thereof, is 50 mg/day, and the dose for riluzole, or a pharmaceutically acceptable salt thereof, is 100 mg/day.

15. Use as claimed in Claim 12, wherein the dose for neramexane, or a pharmaceutically acceptable salt thereof, is 75 mg/day, and the dose for riluzole, or a pharmaceutically acceptable salt thereof, is 100 mg/day.

16. Use as claimed in any preceding claim, wherein the medicament is manufactured for administration of neramexane, or a pharmaceutically acceptable salt thereof, in combination with a Glutamate Release Inhibitor once a day, twice a day (b.i.d.), or three times a day.

17. Use as claimed in any one of Claims 9 to 16, wherein the medicament is manufactured to provide neramexane, or a pharmaceutically acceptable salt thereof, in combination with a Glutamate Release Inhibitor in an immediate release formulation.

18. Use as claimed in any one of Claims 9 to 16, wherein the medicament is manufactured to provide neramexane, or a pharmaceutically acceptable salt thereof, in combination with a Glutamate Release Inhibitor in a modified release formulation.

19. A pharmaceutical composition comprising neramexane, or a pharmaceutically acceptable salt thereof, and a Glutamate Release Inhibitor.

20. The pharmaceutical composition of Claim 19, further comprising a pharmaceutically acceptable carrier.

21. The pharmaceutical composition of Claim 20, which is a solid oral dosage form.

22. The pharmaceutical composition as claimed in any one of Claims 19 to 21, wherein neramexane is employed as its mesylate salt.

23. The pharmaceutical composition as claimed in any one of Claims 19 to 22, wherein the Glutamate Release Inhibitor is selected from riluzole, RP66055, gabapentin, pregabalin, lamotrigine, lubeluzole, fosphenytoin, sipatrigine, MS-153, and FP-0011 and pharmaceutically acceptable salts thereof.

24. The pharmaceutical composition as claimed in Claim 23, wherein the Glutamate Release Inhibitor is selected from riluzole and pharmaceutically acceptable salts thereof.

25. The pharmaceutical composition as claimed in Claim 24, which is appropriately packaged for administration at a dose of 5 to 150 mg/day for neramexane, or a pharmaceutically acceptable salt thereof, and 5 to 150 mg/day for riluzole, or a pharmaceutically acceptable salt thereof.

26. The pharmaceutical composition as claimed in Claim 25, wherein the dose for neramexane, or a pharmaceutically acceptable salt thereof, is 5 mg to 100 mg/day, and the dose for riluzole, or a pharmaceutically acceptable salt thereof, is 5 mg to 100 mg/day.

27. The pharmaceutical composition as claimed in Claim 26, wherein the dose for neramexane, or a pharmaceutically acceptable salt thereof, is 5 mg to 75 mg/day, and the dose for riluzole, or a pharmaceutically acceptable salt thereof, is 5 mg to 100 mg/day.

28. The pharmaceutical composition as claimed in Claim 27, wherein the dose for neramexane, or a pharmaceutically acceptable salt thereof, is 25 mg/day, and the dose for riluzole, or a pharmaceutically acceptable salt thereof, is 100 mg/day.

29. The pharmaceutical composition as claimed in Claim 27, wherein the dose for neramexane, or a pharmaceutically acceptable salt thereof, is 50 mg/day, and the dose for riluzole, or a pharmaceutically acceptable salt thereof, is 100 mg/day.

30. The pharmaceutical composition as claimed in Claim 27, wherein the dose for neramexane, or a pharmaceutically acceptable salt thereof, is 75 mg/day, and the dose for riluzole, or a pharmaceutically acceptable salt thereof, is 100 mg/day.

31. The pharmaceutical composition as claimed in any one of Claims 19 to 30, which is appropriately packaged for administration once a day, twice a day (b.i.d.), or three times a day.

32. The pharmaceutical composition as claimed in any one of Claims 19 to 31, which is provided as an immediate release formulation.

33. The pharmaceutical composition as claimed in any one of Claims 19 to 31, which is provided as a modified release formulation.

34. The pharmaceutical composition as claimed in any one of Claims 19 to 33, for the treatment of neurodegenerative disorders.

35. The pharmaceutical composition as claimed in Claim 34, wherein the neurodegenerative disorder is a motor neuron disease.

36. The pharmaceutical composition as claimed in Claim 35, wherein the motor neuron disease is selected from amyotrophic lateral sclerosis, virus-induced poliomyelitis, lathyrism, primary lateral sclerosis, progressive muscular atrophy, pseudobulbar palsy, progressive bulbar palsy, progressive supranuclear palsy, Kennedy's disease, and spinal muscular atrophy.

37. The pharmaceutical composition as claimed in Claim 36, wherein the motor neuron disease is amyotrophic lateral sclerosis.
